# EUROPÄISCHE PATENTSCHRIFT

(11) **EP 0 489 761 B1**
(45) Veröffentlichungstag und Bekanntmachung des Hinweises auf die Patenterteilung: **07.12.1994**
(21) Anmeldenummer: 90912102.2
(22) Anmeldetag: 20.08.1990
(51) Int. Cl.: C07C 69/33, C07C 69/58, C07C 67/08, C07C 67/60

(54) **VERFAHREN ZUR HERSTELLUNG VON SALZFREIEN ESTERN VON FETTSÄUREN MIT OLIGOGLYCERINEN BZW. MIT ANLAGERUNGSPRODUKTEN VON ETHYLENOXID UND/ODER PROPYLENOXID AN OLIGOGLYCERINE**
PROCESS FOR PREPARING SALT-FREE ESTERS OF FATTY ACIDS WITH OLIGOGLYCERINS OR WITH ADDITION REACTION PRODUCTS OF ETHYLENE OXIDE AND/OR PROPYLENE OXIDE WITH OLIGOGLYCERINS
PROCEDE POUR FABRIQUER DES ESTERS, DEPOURVUS DE SELS, D'ACIDES GRAS AVEC DES OLIGOGLYCERINES OU AVEC DES PRODUITS D'ADDITION D'OXYDE D'ETHYLENE ET/OU D'OXYDE DE PROPYLENE A DES OLIGOGLYCERINES

(30) Priorität: 28.08.1989 DE 3928397
(43) Veröffentlichungstag der Anmeldung: 17.06.1992
(73) Patentinhaber: Henkel Kommanditgesellschaft auf Aktien, 40191 Düsseldorf (DE)
(72) Erfinder: BUNTE, Reinhard, W-4000 Düsseldorf 12 (DE)
(86) Internationale Anmeldenummer: EP9001370
(87) Internationale Veröffentlichungsnummer: WO9103456

(56) Entgegenhaltungen:
- FR-A- 157 466
- US-A- 3 637 774

## Beschreibung

Die Erfindung betrifft ein Verfahren zur Herstellung von salzfreien Estern von Fettsäuren mit Oligoglycerinen bzw. mit Anlagerungsprodukten von Ethylenoxid und/oder Propylenoxid an Oligoglycerine durch Kondensation von Glycerin in Gegenwart von Erdalkalioxiden, -hydroxiden und/oder -carbonaten als Katalysator bei Temperaturen im Bereich von 200 bis 280°C, gegebenenfalls anschließender Anlagerung von Ethylenoxid und/oder Propylenoxid an die erhaltenen Oligoglycerine, Desaktivierung des Katalysators und Veresterung der erhaltenen Oligoglycerine bzw. Anlagerungsprodukte von Ethylenoxid und/oder Propylenoxid an die Oligoglycerine mit Fettsäuren bei Temperaturen im Bereich von 150 bis 270°C.

Ester von Fettsäuren mit Oligoglycerinen bzw. mit Anlagerungsprodukten von Ethylenoxid und/oder Propylenoxid an Oligoglycerine sind bekannte Additive für Nahrungsmittel, Kosmetika und technische Zwecke; sie werden z.B. gemäß US-C 4 397 985 und US-C 3 781 202 als Emulgatoren sowie als Glätte- und Gleitmittel in Faserpräparationen eingesetzt.

Fettsäureoligoglyceride werden im allgemeinen hergestellt, indem man Glycerin in Gegenwart alkalischer oder saurer Katalysatoren kondensiert und die erhaltenen Oligoglycerine mit Fettsäuren verestert. Die dabei im Produkt verbleibenden Katalysatorsalze sind jedoch für viele Anwendungszwecke unerwünscht, so daß es nicht an Versuchen gefehlt hat, diese Verbindungen in salz- bzw. elektrolytfreier Form herzustellen. So beschreibt die FR-C 1 575 466 ein Verfahren, bei dem zunächst Glycerin in Gegenwart von Erdalkalioxiden, -hydroxiden und/oder -carbonaten polykondensiert wird; die in den erhaltenen Oligoglycerinen enthaltenen Katalysatoren werden anschließend vor der Veresterung mit Ionenaustauschern entfernt. Ein Verfahren zur Herstellung von salzfreien Oligoglycerinen, das ebenfalls mit Ionenaustauschern arbeitet, ist aus der DE-A 34 10 520 bekannt.

Verfahren zur Herstellung von Estern von Fettsäuren mit Oligoglycerinen bzw. mit Anlagerungsprodukten von Ethylenoxid und/oder Propylenoxid an Oligoglycerine, bei denen die eingesetzten Oligoglycerine bzw. Anlagerungsprodukte von Ethylenoxid und/oder Propylenoxid an Oligoglycerine, d.h. Oligoglycerin-(poly)ethoxylate bzw. -(poly)propoxylate, von den in ihnen enthaltenen Salzen durch Ionenaustauschverfahren gereinigt werden, sind naturgemäß aufwendig durchzuführen. Somit ist die Erfindung auf ein Verfahren gerichtet, bei dem salzfreie Ester von Fettsäuren mit Oligoglycerinen bzw. mit Anlagerungsprodukten von Ethylenoxid und/oder Propylenoxid an Oligoglycerine ohne aufwendige Trennoperationen für in ihnen enthaltene Salze bzw. Elektrolyte erhalten werden können.

Diese Aufgabe wird erfindungsgemäß dadurch gelöst, daß man dem bei der Kondensation und gegebenenfalls der Umsetzung mit Ethylenoxid und/oder Propylenoxid erhaltenen Reaktionsgemisch eine Säure in einer den Katalysatoren äquivalenten Menge zusetzt und die gebildeten schwerlöslichen Erdalkalisalze nach der Veresterung abtrennt. Dabei wird ein auf einfache Weise im wesentlichen salz- bzw. elektrolytfreies Endprodukt erhalten. Ein weiterer Vorteil ist, daß das Verfahren der Erfindung als "Ein-Topf-Verfahren" durchgeführt werden kann.

Das Verfahren der Erfindung eignet sich zur Herstellung von salzfreien Estern von Fettsäuren mit Oligoglycerinen bzw. mit Anlagerungsprodukten von Ethylenoxid und/oder Propylenoxid an Oligoglycerine, deren Fettsäurereste von Fettsäuren natürlicher und/oder synthetischer Herkunft gebildet sind. Besonders bevorzugt sind Reste von Fettsäuren mit 6 bis 22 Kohlenstoffatomen einschließlich technischen Gemischen derselben, wie sie aus natürlichen, nachwachsenden Rohstoffen tierischer und/oder pflanzlicher Herkunft zugänglich sind; typische Beispiele für derartige Fettsäuren sind Capron-, Capryl-, Caprin-, Laurin-, Myristin-, Palmitin-, Stearin-, Arachin-, Behen-, Palmitolein-, Öl-, Gadolein-, Eruca-, Ricinol-, Linol- und Linolensäure. Es können jedoch auch Reste von Fettsäuren mit mehr als 22 Kohlenstoffatomen, z.B. Montansäuren, in den erfindungsgemäß erhaltenen Verbindungen vorliegen.

Die in dem Verfahren der Erfindung einsetzbaren Katalysatoren für die Kondensation des Glycerins und gegebenenfalls der Ethoxylierung und/oder Propoxylierung der erhaltenen Oligoglycerine sind Oxide, Hydroxide und/oder Carbonate von Erdalkalimetallen wie Calcium, Strontium und Barium; die genannten Verbindungen des Calciums sind hier bevorzugt. Im Verlauf der Kondensation des Glycerins wird ein Gemisch von Oligoglycerinen unterschiedlichen Kondensationsgrades erhalten; die gebildeten Oligoglycerine weisen im Zahlenmittel 2 bis 20, insbesondere 2 bis 10 und bevorzugt 2 bis 5 Glycerineinheiten auf. Typische Vertreter für hier angesprochene Oligoglycerine sind z.B. Di-, Tri-, Tetra-, Penta-, Hexa-, Hepta-, Octa- und Decaglycerin. Die gegebenenfalls vorgesehene anschließende Anlagerung von Ethylenoxid und/oder Propylenoxid an die Oligoglycerine erfolgt in Anwesenheit der vorgenannten Katalysatoren unter üblichen Bedingungen bei erhöhten Temperaturen und Druck. Dabei werden bevorzugt 1 bis 20 Mol Ethylenoxid und/oder Propylenoxid pro Mol Oligoglycerin angelagert. Falls dabei gemischte Anlagerungsprodukte von Ethylenoxid und Propylenoxid an Oligoglycerine gebildet werden sollen, können Ethylenoxy- und Propylenoxygruppen in random- oder block-Verteilung an die Oligoglycerine angelagert werden.

Gemäß dem Verfahren der Erfindung setzt man dem bei der Kondensation des Glycerins und gegebenenfalls der Anlagerung von Ethylenoxid und/oder Propoxylenoxid an die erhaltenen Oligoglycerine erhaltenen Reaktionsgemisch eine Säure in einer den Katalysatoren äquivalenten Menge zu, d.h. in einer Menge, die zur Überführung der Katalysatoren in ihre schwerlöslichen Erdalkalisalze erforderlich ist. Die Salze verbleiben während der nachfolgendenden Veresterung in dem Reaktionsgemisch und werden erst abgetrennt, nachdem die Veresterung abgeschlossen ist. Bevorzugte Säuren sind Schwefel- und/oder Phosphorsäure, die mit den Katalysatoren zu in dem bei der Veresterung erhaltenen Reaktionsgemisch besonders schwerlöslichen schwefel- und/oder phosphorsauren Erdalkalisalzen reagieren. Speziell bevorzugt ist Schwefelsäure, die mit den Katalysatoren besonders einfach abtrennbare Erdalkalisulfate ergibt.

Gemäß einer vorteilhaften Ausführungsform der Erfindung kondensiert man das Glycerin in Gegenwart der genannten Katalysatoren innerhalb von 5 bis 24 Stunden unter fortlaufender Entfernung des Kondensationswassers.

Gemäß einer weiteren vorteilhaften Ausführungsform der Erfindung setzt man dem bei der Kondensation und gegebenenfalls der Anlagerung von Ethylenoxid und/oder Propylenoxid an die erhaltenen Oligoglycerine gebildeten Reaktionsgemisch bei Temperaturen von höchstens 90°C zur Desaktivierung der Katalysatoren eine wäßrige Schwefelsäure- und/oder Phosphorsäurelösung zu; das nunmehr in dem Reaktionsgemisch enthaltene Wasser wird bevorzugt vor der Veresterung mit den Fettsäuren entfernt. Es ist jedoch auch möglich, das dabei eingebrachte Wasser erst im Verlauf der Veresterung zu entfernen.

Gemäß einer weiteren vorteilhaften Ausführungsform der Erfindung wird das im Verlauf der Veresterung gebildete Wasser fortlaufend entfernt.

Gemäß einer weiteren vorteilhaften Ausführungsform der Erfindung filtriert man die in dem bei der Veresterung gebildeten Reaktionsgemisch enthaltenen schwerlöslichen Erdalkalisalze ab. Es sind jedoch auch andere Trennmethoden möglich, so kann z.B. die Abtrennung auch in Zentrifugen erfolgen.

Gemäß einer weiteren vorteilhaften Ausführungsform der Erfindung führt man die Stufen der Kondensation, Anlagerung von Ethylenoxid und/oder Propylenoxid und der Veresterung in Abwesenheit von Lösemitteln durch; lediglich in der Stufe der Desaktivierung des Kondensationskatalysators kann mit einem Lösemittel, d.h. mit Wasser, gearbeitet werden, das jedoch vor oder in der nachfolgenden Stufe der Veresterung oder im Verlauf derselben entfernt wird.

Das Verfahren der Erfindung ist insbesondere geeignet zur Herstellung von mit Fettsäuren mit 6 bis 22 Kohlenstoffatomen einschließlich Gemischen derselben partiell veresterten, 2 bis 20 Glycerineinheiten und gegebenenfalls 1 bis 20, insbesondere 1 bis 10 angelagerte Ethylenoxy- bzw. Propylenoxygruppen enthaltenden Oligoglycerinen mit Säurezahlen von weniger als 1 und Salzgehalten von weniger als 500, insbesondere von weniger als 50 ppm. Der vorgenannte Begriff "partiell verestert" bedeutet, daß die erfindungsgemäß erhaltenen Ester von Fettsäuren mit Oligoglycerinen bzw. mit Anlagerungsprodukten von Ethylenoxid und/oder Propylenoxid an Oligoglycerine mindestens einen Fettsäurerest und mindestens eine freie OH-Gruppe aufweisen.

In Anbetracht der bei der Kondensation und der Veresterung erforderlichen Reaktionstemperaturen sowie der dabei gegebenen Oxidationsempfindlichkeit der Reaktanden, insbesondere bei der Veresterung mit ungesättigten Fettsäuren, ist es zweckmäßig, diese Verfahrensschritte unter Schutzgas durchzuführen; selbstverständlich gilt dies auch für den gegebenenfalls vorgesehenen Schritt der Ethoxylierung und/oder Propoxylierung der Oligoglycerine.

Das Verfahren der Erfindung wird im folgenden anhand von bevorzugten Ausführungsbeispielen näher erläutert.

### Beispiel 1.

### Herstellung eines Partialesters aus einer technischen C_{8/10}-Fettsäure und Diglycerin im Molverhältnis von 1 : 1.

1,84 kg Glycerin (20,0 mol) wurden in Gegenwart von 37 g Ca(OH)₂ (0,5 mol) 9,5 Stunden unter Stickstoff bei 230 bis 240°C kondensiert. Im Verlauf der Reaktion destillierten 210 ml Kondensationswasser über.

Anschließend wurden dem Reaktionsgemisch 200 ml einer 25 %-igen wäßrigen Schwefelsäurelösung (0,5 mol) bei einer Temperatur von 40 bis 50°C zugesetzt; das erhaltene Gemisch wurde bei 120°C unter Entfernung des Wassers getrocknet.

Anschließend wurde das Gemisch mit 1,52 kg einer technischen Vorlauffettsäure (9,8 mol; Kettenlängenverteilung: 2 % C₆, 60 % C₈, 35 % C₁₀, 3 % C₁₂) versetzt und 8 Stunden bei 180 bis 200°C unter Stickstoff erhitzt. Das gebildete Wasser wurde fortlaufend - gegen Ende der Reaktion im Vakuum - entfernt. Das erhaltene Produkt wurde nach dem Abkühlen auf 90 bis 100°C zur Entfernung von Calciumsulfat filtriert und wies die folgenden Kennzahlen auf:
Hydroxylzahl 528
Verseifungszahl 209
Säurezahl 0,9
Ca-Gehalt: < 50 ppm.

Die Wiederholung des vorgenannten Beispiels unter Verwendung von 0,5 Mol Phosphorsäure anstelle von Schwefelsäure ergab ein ähnliches Produkt, wobei das Abfiltrieren des Calciumphosphats jedoch eine längere Zeit erforderte.

### Beispiel 2.

### Partialester aus Triglycerin und Ölsäure (Molverhältnis: 1 : 1; Triglycerin-monooleat).

55,24 kg Glycerin (600 mol) wurden in Gegenwart von 1,05 kg Ca(OH)₂ (14,91 mol) 11,5 h im Stickstoffstrom bei 230 bis 240°C kondensiert. Dabei entstanden ca. 9 l Kondensationswasser, das fortlaufend entfernt wurde. Nach Zugabe von 1,46 kg Schwefelsäure (14,90 Mol) in 4,4 l Wasser bei 60°C, gefolgt von einer Entfernung des dabei eingebrachten Wassers, wurde das erhaltene Oligoglycerin mit 55,6 kg einer technischen Ölsäure (200 mol; Kettenlängenverteilung: 5 % C₁₆, 1 % C₁₇, 2 % C₁₈ (gesättigt), 67 % C₁₈ (einfach ungesättigt), 12 % C₁₈ (zweifach ungesättigt), 1 % C₁₈ (dreifach ungesättigt), 2 % > C₁₈) bei 180 bis 200°C 9 Stunden lang unter fortlaufender Entfernung des Reaktionswassers verestert. Das Produkt wurde nach dem Abkühlen auf etwa 100°C filtriert und wies die folgenden Kennzahlen auf:
Hydroxylzahl 333
Verseifungszahl 156
Säurezahl 0,5
Ca-Gehalt: < 50 ppm.

### Beispiel 3.

### Herstellung eines Partialesters aus einer technischen C₈/C₁₀-Fettsäure und einem Anlagerungsprodukt von 2 Mol Propylenoxid an 1 Mol Diglycerin (Molverhältnis von Fettshure zu Diglycerin-bispropoxylat ca. 2:1).

Analog zu der in Beispiel 1 beschriebenen Weise wurden zunächst 747 g (4,5 mol) eines Diglycerins unter Verwendung von 16,7 g Ca(OH)₂ (0,23 mol) als Katalysator hergestellt. Das erhaltene Reaktionsgemisch wurde mit 522 g (9 mol) Propylenoxid in einem Autoklaven bei 160°C umgesetzt. Die Hydroxylzahl des erhaltenen Bispropoxylats betrug 816.

564 g (2 mol) des erhaltenen Bispropoxylats wurden mit einer dem darin enthaltenen Ca(OH)₂ äquivalenten Schwefelsäuremenge (9,9 g) in 40 ml Wasser versetzt und anschließend mit 660 g (4,2 mol) einer C_{8/10}-Fettsäure mit der in Beispiel 1 angegebenen Zusammensetzung und wie dort beschrieben umgesetzt.

Nach der Entfernung des Calciumsulfats durch Filtration wies das Produkt eine Hydroxylzahl von 193, eine Verseifungszahl von 219 und eine Säurezahl von 0,5 auf; der Calciumgehalt lag unter 50 ppm.

### Beispiel 4.

### Herstellung eines Partialesters aus einer technischen Ölsäure und Heptaglycerin im Molverhältnis von ca. 5:1.

Analog zu der in Beispiel 1 beschriebenen Weise wurden zunächst 56,53 kg (614 mol) Glycerin mit 1,41 kg (19,0 mol) Ca(OH)₂ 23,5 h bei 230 bis 250°C kondensiert; in dieser Zeit waren 13,5 l (75 mol) Wasser abdestilliert. Aus der Menge des Kondensatwassers ergab sich, daß das Reaktionsgemisch Heptaglycerin als Hauptprodukt enthielt.

Das erhaltene Reaktionsgemisch wurde analog zu der in Beispiel 1 beschriebenen Arbeitsweise zunächst mit 1,86 kg (19,0 mol) Schwefelsäure in 5,6 l Wasser versetzt und anschließend mit 66,72 kg (240 mol) einer technischen Ölsäure der in Beispiel 2 angegebenen Zusammensetzung verestert. Nach dem Abfiltrieren des Calciumsulfats wies das Reaktionsprodukt eine Hydroxylzahl von 217, eine Verseifungszahl von 169, eine Säurezahl von 0,8 sowie einen Ca-Gehalt von weniger als 50 ppm auf.

## Patentansprüche

1. Verfahren zur Herstellung von salzfreien Estern von Fettsäuren mit Oligoglycerinen bzw. mit Anlagerungsprodukten von Ethylenoxid und/oder Propylenoxid an Oligoglycerine durch Kondensation von Glycerin in Gegenwart von Erdalkalioxiden, -hydroxiden und/oder -carbonaten als Katalysator bei Temperaturen im Bereich von 200 bis 280°C, gegebenenfalls anschließender Anlagerung von Ethylenoxid und/oder Propylenoxid an die erhaltenen Oligoglycerine, Desaktivierung des Katalysators und Veresterung der erhaltenen Oligoglycerine bzw. Anlagerungsprodukte von Ethylenoxid und/oder Propylenoxid an die Oligoglycerine mit Fettsäuren bei Temperaturen im Bereich von 150 bis 270°C, dadurch gekennzeichnet, daß man dem bei der Kondensation und gegebenenfalls der Umsetzung mit Ethylenoxid und/oder Propylenoxid erhaltenen Reaktionsgemisch eine Säure in einer dem Katalysator äquivalenten Menge zusetzt und die mit der Säure gebildeten schwerlöslichen Erdalkalisalze nach der Veresterung abtrennt.

2. Verfahren nach Anspruch 1, dadurch gekennzeichnet, daß man bei der Veresterung Fettsäuren mit 6 bis 22 Kohlenstoffatomen bzw. technische Gemische derselben verwendet.

3. Verfahren nach Anspruch 1 oder 2, dadurch gekennzeichnet, daß man das Glycerin innerhalb von 5 bis 24 Stunden unter fortlaufender Entfernung des Kondensationswassers kondensiert.

4. Verfahren nach mindestens einem der Ansprüche 1 bis 3, dadurch gekennzeichnet, daß man dem bei der Kondensation des Glycerins und gegebenenfalls der Anlagerung von Ethylenoxid und/oder Propylenoxid erhaltenen Reaktionsgemisch Schwefel-und/oder Phosphorsäure zusetzt.

5. Verfahren nach mindestens einem der Ansprüche 1 bis 4, dadurch gekennzeichnet, daß man dem bei der Kondensation des Glycerins und gegebenenfalls der Anlagerung von Ethylenoxid und/oder Propylenoxid erhaltenen Reaktionsgemisch bei Temperaturen von höchstens 90°C wäßrige Schwefelsäure- und/oder Phosphorsäurelösungen zusetzt.

6. Verfahren nach mindestens einem der Ansprüche 1 bis 5, dadurch gekennzeichnet, daß man das in dem bei der Kondensation des Glycerins und gegebenenfalls der Anlagerung von Ethylenoxid und/oder Propylenoxid erhaltenen und mit Schwefel-und/oder Phosphorsäure versetzten Reaktionsgemisch enthaltene Wasser vor der Veresterung mit den Fettsäuren entfernt.

7. Verfahren nach mindestens einem der Ansprüche 1 bis 6, dadurch gekennzeichnet, daß man das bei der Veresterung gebildete Wasser fortlaufend entfernt.

8. Verfahren nach mindestens einem der Ansprüche 1 bis 7, dadurch gekennzeichnet, daß man die in dem bei der Veresterung gebildeten Reaktionsgemisch enthaltenen schwerlöslichen Erdalkalisalze abfiltriert.

9. Verfahren nach mindestens einem der Ansprüche 1 bis 8, dadurch gekennzeichnet, daß man die Stufen der Kondensation und der Veresterung in Abwesenheit von Lösemitteln durchführt.

10. Verfahren nach mindestens einem der Ansprüche 1 bis 9 zur Herstellung von mit Fettsäuren mit 6 bis 22 Kohlenstoffatomen partiell veresterten, 2 bis 20 Glycerineinheiten und gegebenenfalls 1 bis 20 angelagerte Ethylenoxy- bzw. Propylenoxygruppen enthaltenden Oligoglycerinen mit Säurezahlen von weniger als 1 und Salzgehalten von weniger als 500, insbesondere von weniger als 50 ppm.

## Claims

1. A process for the production of salt-free esters of fatty acids with oligoglycerols or with addition products of ethylene oxide and/or propylene oxide with oligoglycerols by condensation of glycerol in the presence of alkaline earth metal oxides, hydroxides and/or carbonates as catalyst and at temperatures in the range from 200 to 280°C, optionally subsequent addition of ethylene oxide and/or propylene oxide onto the oligoglycerols obtained, deactivation of the catalyst and esterification of the resulting oligoglyercols or addition products of ethylene oxide and/or propylene oxide with the oligoglycerols with fatty acids at temperatures in the range from 150 to 270°C, characterized in that an acid in a quantity equivalent to the catalyst is added to the reaction mixture obtained in the condensation step and in the optional reaction with ethylene oxide and/or propylene oxide and the poorly soluble alkaline earth metal salts formed with the acid are separated off after the esterification step.

2. A process as claimed in claim 1, characterized in that C₆₋₂₂ fatty acids or technical mixtures thereof are used in the esterification step.

3. A process as claimed in claim 1 or 2, characterized in that the glycerol is condensed for 5 to 24 hours with continuous removal of the water of condensation.

4. A process as claimed in at least one of claims 1 to 3, characterized in that sulfuric and/or phosphoric acid is/are added to the reaction mixture obtained in the condensation of the glycerol and in the optional addition of ethylene oxide and/or propylene oxide.

5. A process as claimed in at least one of claims 1 to 4, characterized in that aqueous sulfuric acid and/or phosphoric acid solutions are added to the reaction mixture obtained in the condensation of the glycerol and in the optional addition of ethylene oxide and/or propylene oxide at temperatures of at most 90°C.

6. A process as claimed in at least one of claims 1 to 5, characterized in that the water present in the reaction mixture obtained in the condensation of the glycerol and in the optional addition of ethylene oxide and/or propylene oxide, to which sulfuric acid and/or phosphoric acid have been added, is removed before the esterification step with the fatty acids.

7. A process as claimed in at least one of claims 1 to 6, characterized in that the water formed in the esterification step is continuously removed.

8. A process as claimed in at least one of claims 1 to 7, characterized in that the poorly soluble alkaline earth metal salts present in the reaction mixture formed during the esterification step are filtered off.

9. A process as claimed in at least one of claims 1 to 8, characterized in that the condensation and esterification steps are carried out in the absence of solvents.

10. A process as claimed in at least one of claims 1 to 9 for the production of oligoglycerols partly esterified with C₆₋₂₂ fatty acids and containing 2 to 20 glycerol units and optionally 1 to 20 added ethyleneoxy or propyleneoxy groups for acid values of less than 1 and salt contents of less than 500 ppm and preferably less than 50 ppm.

## Revendications

1. Procédé pour produire des esters, dépourvus de sels, d'acides gras avec des oligoglycérines ou avec des produits d'addition d'oxyde d'éthylène et/ou d'oxyde de propylène à des oligoglycérines par condensation de glycérine en présence d'oxydes, d'hydroxydes, et/ou de carbonates alcalino-terreux comme catalyseur à des températures dans la gamme de 200 à 280°C, éventuellement par addition successive d'oxyde d'éthylène et/ou d'oxyde de propylène aux oligoglycérines obtenues, désactivation du catalyseur et estérification des oligoglycérines obtenues ou des produits d'addition d'oxyde d'éthylène et/ou d'oxyde de propylène aux oligoglycérines avec des acides gras à des températures dans la gamme de 150 à 270°C, caractérisé en ce qu'on ajoute au mélange réactionnel obtenu lors de la condensation et éventuellement lors de la transformation avec de l'oxyde d'éthylène et/ou de l'oxyde de propylène, un acide en une quantité équivalente à celle du catalyseur et on sépare après l'estérification les sels alcalinoterreux difficilement solubles formés avec l'acide.

2. Procédé selon la revendication 1, caractérisé en ce qu'on utilise pour l'estérification des acides gras avec de 6 à 22 atomes de carbone ou des mélanges techniques de ceux-ci mêmes.

3. Procédé selon les revendications 1 ou 2, caractérisé en ce qu'on condense la glycérine pendant 5 à 24 heures en enlevant continuellement l'eau de condensation.

4. Procédé selon une au moins des revendications 1 à 3, caractérisé en ce qu'on ajoute au mélange réactionnel obtenu lors de la condensation de la glycérine et éventuellement de l'addition d'oxyde d'éthylène et/ou d'oxyde de propylène, de l'acide sulfurique et/ou de l'acide phosphorique.

5. Procédé selon l'une au moins des revendications 1 à 4, caractérisé en ce qu'on ajoute au mélange réactionnel obtenu lors de la condensation de la glycérine et éventuellement de l'addition d'oxyde d'éthylène et/ou d'oxyde de propylène à des températures d'au maximum 90°C, des solutions aqueuses d'acide sulfurique et/ou d'acide phosphorique.

6. Procédé selon l'une au moins des revendications 1 à 5, caractérisé en ce qu'on élimine avant l'estérification avec les acides gras l'eau contenue dans le mélange réactionnel obtenu lors de la condensation de la glycérine et éventuellement de l'addition d'oxyde d'éthylène et/ou d'oxyde de propylène et mélangé à de l'acide sulfurique et/ou phosphorique.

7. Procédé selon l'une au moins des revendications 1 à 6, caractérisé en ce qu'on élimine en continu l'eau formée lors de l'estérification.

8. Procédé selon l'une au moins des revendications 1 à 7, caractérisé en ce qu'on élimine par filtration des sels alcalino-terreux difficilement solubles contenus dans le mélange réactionnel formé lors de l'estérification.

9. Procédé selon l'une au moins des revendications 1 à 8, caractérisé en ce qu'on réalise les étapes de la condensation et de l'estérification en l'absence de solvants.

10. Procédé selon l'une au moins des revendications 1 à 9, destiné à produire des oligoglycérines estérifiées partiellement avec des acides gras ayant de 6 à 22 atomes de carbone, contenant 2 à 20 unités de glycérine et éventuellement 1 à 20 groupes d'oxyde d'éthylène ou d'oxyde de propylène ajoutés et ayant des indices d'acide inférieurs à 1 et des teneurs en sels inférieures à 500, de préférence inférieures à 50 ppm.
